# EUROPEAN PATENT SPECIFICATION

(11) **EP 4 003 283 B1**
(45) Date of publication and mention of the grant of the patent: **30.08.2023**
(21) Application number: 20746650.9
(22) Date of filing: 29.07.2020
(51) Int. Cl.: A61K 31/05, A61K 8/34, A61P 17/10, A61Q 19/00, A61K 31/352, A61K 8/9789, A61K 31/357, A61K 31/7024, A61K 36/185, A61K 36/23, A61K 36/28

(54) **TREATMENT OF INFLAMMATORY SKIN CONDITIONS**
BEHANDLUNG VON ENTZÜNDLICHEN HAUTERKRANKUNGEN
TRAITEMENT D'AFFECTIONS CUTANÉES INFLAMMATOIRES

(30) Priority: 29.07.2019 EP 19188768
(43) Date of publication of application: 01.06.2022
(73) Proprietor: Echo Pharmaceuticals B.V., 2333 CG Leiden (NL)
(72) Inventor: VERBAKEL, Joost, 2333 CG Leiden (NL); ROZENBLAT, Sharon, 2333 CG Leiden (NL)
(74) Representative: Nederlandsch Octrooibureau
(86) International application number: PCT/EP2020/071345
(87) International publication number: WO 2021/018936

(56) References cited:
- WO-A1-2018/124369
- US-A1- 2011 081 433
- US-A1- 2018 021 247

## Description

### TECHNICAL FIELD OF THE INVENTION

The present invention relates to a topical formulation according to Claim 1 for use in the treatment of inflammatory skin conditions, such as acne vulgaris, said treatment comprising topically administrating a formulation comprising (i) cannabidiol and (ii) flavonolignans selected from silibinin, isosilibinin, silicristin, silidianin and combinations thereof. These flavonolignans may suitably be provided by an extract of *Silybum marianum.*

The invention further relates to a topical formulation comprising:
- cannabidiol;
- flavonolignans selected silibinin, isosilibinin, silicristin, silidianin and combinations thereof, the concentrations of these flavonolignans being calculated as silibinin;
- triterpenes selected from asiaticoside, madecassoside, asiatic acid, madecassic acid and combinations thereof.

The triterpenes can be provided, for instance, by an extract of *Centella asiatica.*

### BACKGROUND OF THE INVENTION

Inflammatory skin diseases are the most common problem in dermatology. They come in many forms, from occasional rashes accompanied by skin itching and redness, to chronic conditions such as dermatitis (eczema), rosacea, seborrheic dermatitis, and psoriasis. Skin inflammation can be characterized as acute or chronic.

Acute inflammation can result from exposure to UV radiation (UVR), ionizing radiation, allergens, or to contact with chemical irritants (soaps, hair dyes, etc.). This type of inflammation is typically resolved within 1 to 2 weeks with little accompanying tissue destruction. In contrast, chronic inflammation results from a sustained immune cell mediated inflammatory response within the skin itself. This inflammation is long lasting and can cause significant and serious tissue destruction.

The process of skin inflammation is complex and is still not completely understood. When the skin is exposed to a "triggering" stimulus, such as UV radiation, an irritant (e.g. soaps or fragrances), or to allergens, the cells in the skin produce a variety of inflammatory mediators called cytokines and chemokines. These "inflammatory messengers" bind to specific receptors on target cells and stimulate the production of additional inflammatory signalling mediators. Some of these cause vasodilation while others activate nerve cells. Still other cytokines cause immune cells to leave the blood and migrate into the skin where they then produce more inflammatory mediators, as well as enzymes, free radicals, and chemicals that damage the skin. The end result of the initial triggering event is the amplification of a large inflammatory response that, while designed to help the skin fight infection from invading bacteria, actually causes considerable damage to the skin.

The most effective and commonly used prescription drugs for treating inflammation are the corticosteroids, particularly the glucocorticoid related steroids. They are very effective for many forms of eczema, including atopic dermatitis, allergic contact dermatitis, seborrheic dermatitis (in concert with an anti-fungal agent), and are fairly effective in ameliorating the symptoms of psoriasis. They are not particularly effective, however, in treating acute inflammation, like UVR induced sunburn, which is not primarily an immune cell driven inflammatory response. Also, both systemic and topical corticosteroids can exacerbate or precipitate acne. Corticosteroids can be used topically or orally.

While current treatment regimens for most inflammatory skin diseases are dominated by corticosteroids and antibiotics, these are typically used for only short periods of time because they exert negative side effects on skin.

Acne vulgaris is a follicular disease characterized by pilosebaceous inflammations such as comedones, papules, pustules, cysts and nodules. Major progressive factors in the development acne include hyperkeratosis of the follicular epithelium, increased sebum production, and proliferation of *Propionibacterium acnes.* These factors are primarily responsible for hyperkeratosis of the follicle lining, including retention of keratin and sebum, as well as the free fatty acid by-products of *P*. *acnes* metabolization which can lead to inflamed acne papules and pustules.

Although acne may also be influenced by exogeneous and hormonal factors, research has been centered around eliminating *P*. *acnes,* the most common cause of inflammation. To date, the pathogenesis of acne is not fully understood, and there is currently no cure for the disease. Many systemic and topical medications, such as tetracycline, have been used to manage and control acne. None, however, is universally successful.

Acne is widely considered a chronic skin disease that primarily affects individuals going through puberty, with a prevalence among this population of more than 80 percent. However, although acne is principally a disorder of adolescence, current research indicates that the prevalence of adult patients with acne, especially among women, is increasing.

Olah et al. (Cannabidiol exerts sebostatic and antiinflammatory effects on human sebocytes. J Clin Invest 2014; 124: 3713-3724) explored the effects of (-)-cannabidiol (CBD) on human sebaceous gland function and determined that CBD behaves as a highly effective sebostatic agent. Administration of CBD to cultured human sebocytes and human skin organ culture inhibited the lipogenic actions of various compounds, including arachidonic acid and a combination of linoleic acid and testosterone, and suppressed sebocyte proliferation via the activation of transient receptor potential vanilloid-4 (TRPV4) ion channels. Activation of TRPV4 interfered with the prolipogenic ERK1/2 MAPK pathway and resulted in the downregulation of nuclear receptor interacting protein-1 (NRIP1), which influences glucose and lipid metabolism, thereby inhibiting sebocyte lipogenesis. CBD also exerted complex anti-inflammatory actions that were coupled to A2a adenosine receptor-dependent upregulation of tribbles homolog 3 (TRIB3) and inhibition of the NF-_{K}B signaling. The authors conclude that thee findings suggest that, due to the combined lipostatic, anti proliferative, and antiinflammatory effects, CBD has potential as a promising therapeutic agent for the treatment of acne vulgaris

US 2018/0263952 describes a method of treating an inflammatory skin disease comprising administering one or more of the cannabinoids selected from the group consisting of: cannabidiol (CBD), cannabidiolic acid (CBDA). cannabidivarin (CBDV). Cannabigerol (CBG). cannabigervarin (CBGV) and tetrahydrocannabivarin (THCV) to a subject.

US 2019/0111093 describes a topical composition comprising caprylic/capric triglyceride, aloe barbadensis leaf juice, stearic acid, glycerol monostearate, trietanolamine, vitamin E, water, olive europaea fruit oil, glycerol, acrylates/C₁₀₋₃₀ alkyl acrylate crosspolymer, calendula officinalis flower extract, phenoxyethanol, decarboxylated Cannabis sativa extract (0.25% of CBD) and non-carboxylated Cannabis sativa extract (0.25% CBDA), where cannabis sativa extract comprises about 80% of CBDA from the total cannabinoids, and THC is less than 0.1 %.The topical composition can be used for treatment of skin discomfort caused by skin inflammation, atopic dermatitis, psoriasis, urticaria, radiotherapy induced atopic dermatitis, UV and/or oxidation skin and/or thirst or second degree burn and damage associated with skin moisture disbalance.

US 2016/0235661 describes topical compositions comprising (i) hemp oil containing 3-24 wt.% cannabigerol and 1-10 wt.% cannabidiol and (ii) at least one anti-oxidant selected from the group consisting of *Vitis vinifera* extract, tocopherol, tocopherol derivatives, betacarotene, anthocyans, catechins, quercetins, genistein extract, acetyl hexapeptide-3, *Silybum marianum* fruit extract, and silymarin.

US 2011/0151032 describes a method of treating or ameliorating an indication of nonmucosal topical tissue comprising periodically applying to such disease affected tissue a composition comprising an effective amount of an appropriate composition of herbal bioactive comprising active(s) of one or more of *Sambucus nigra, Centella asiatica* or *Echinacea purpurea,* and an effective amount of a quaternary ammonium surfactant.

CN 108524414 describes an aqueous cosmetic composition for topical application (a mask), comprising water, humectant, thickening agent, preservative, solubilizers, cannabidiol, propolis extract, ginseng extract, ginseng essential oil, collagen, *Centella asiatica* extract, and rosmarinic acid.

CN 109419633 describes a sustained release system for topical application, comprising cannabis leaf extract. The examples describe systems that contain *Centella asiatica* extract.

### SUMMARY OF THE INVENTION

The present invention provides a topical formulation according to Claim 1 for use in a method of treating or preventing an inflammatory skin condition, said method comprising topically administrating a formulation comprising (i) cannabidiol and (ii) flavonolignans selected from silibinin, isosilibinin, silicristin, silidianin and combinations thereof.

It was unexpectedly found that the combination of cannabidiol and the aforementioned flavonolignans is highly effective against inflammatory skin conditions, such as acne vulgaris. Although the inventors do not wish to be bound by theory, it is believed that this high effectiveness is associated with a synergistic interaction between cannabidiol and the flavonolignans.

The invention further relates to a topical formulation comprising:
- cannabidiol;
- flavonolignans selected silibinin, isosilibinin, silicristin, silidianin and combinations thereof, the concentrations of these flavonolignans being calculated as silibinin;
- triterpenes selected from asiaticoside, madecassoside, asiatic acid, madecassic acid and combinations thereof.

The inclusion of the aforementioned triterpenes further enhances the effectiveness of the combination of cannabidiol and triterpenes in the treatment of inflammatory skin conditions.

Cannadibiol, the flavonolignans as well as the triterpenes can suitably be provided in the form of plant extracts. Cannabidiol can be extracted from *Cannabis,* the flavonolignans silibinin, isosilibinin, silicristin and silidianin from *Silybum marianum* (fruit without pappus) and the triterpenes asiaticoside, madecassoside, asiatic acid and madecassic acid from *Centella asiatica* (leaf).

### DETAILED DESCRIPTON OF THE INVENTION

A first aspect of the invention relates to a topical formulation for use in a method of treating or preventing an inflammatory skin condition, said method comprising topically administrating a formulation comprising (i) cannabidiol and (ii) flavonolignans selected from silibinin, isosilibinin, silicristin, silidianin and combinations thereof.

Examples of inflammatory skin conditions that may suitably be treated in accordance with the present invention include acne, seborrhea, rosacea, perioral dermatitis, atopic dermatitis, psoriasis, corticosteroid-induced acneiform lesions and oily skin. Preferably, the inflammatory skin condition that is treated in accordance with the present invention is a sebaceous gland disorder selected from acne, seborrhea, rosacea, perioral dermatitis, corticosteroid-induced acneiform lesions and oily skin. The present treatment is particularly suitable for treating acne vulgaris, including adolescence acne and adult acne.

The method present treatment preferably comprises topical administration of the formulation to the skin of a human subject. According to a particularly preferred embodiment, the formulation the treatment comprises topical administration to the face, the upper part of the chest or the back of a human subject.

The formulation that is employed in the treatment according to the present invention preferably contains 0.1-3 wt.% cannabidiol, more preferably 0.3-2 wt.% cannibidiol and most preferably 0.5-1 wt.% cannabidiol.

The flavonolignans selected from silibinin, isosilibinin, silicristin, silidianin and combinations thereof are preferably present in the formulation in a concentration of 0.05-2 wt.%, more preferably 0.05-1 wt.% and most preferably 0.25-1 wt.%, the concentrations of these flavonolignans being calculated as silibinin.

Silibinin is preferably contained in the formulation in a concentration of 0.01-1.5 wt.%, more preferably of 0.03-1.2 wt.% and most preferably of 0.1-1 wt.%.

In another preferred embodiment, the formulation contains 0.001-1.5 wt.%, more preferably 0.01-1 wt.% and most preferably 0.05-0.5 wt.% of silicristin and/or silidianin, the concentrations of these flavonolignans being calculated as silibinin.

Cannabidiol and the aforementioned flavonolignans (concentration of the flavonolignans being calculated as silibinin) are preferably present in a weight ratio of 1:6 to 6:1, more preferably in a weight ratio of 1:4 to 4:1.

The effectiveness of the present formulation against inflammatory skin conditions may be further enhanced by the additional inclusion of triterpenes selected from asiaticoside, madecassoside, asiatic acid, madecassic acid and combinations thereof. These triterpenes are preferably present in the formulation in a concentration of 0.025-3 wt.%, more preferably in a concentration of 0.05-2 wt.% and most preferably in a concentration of 0.1-1 wt.%.

In a preferred embodiment, the formulation contains the triterpene asiaticoside. More preferably, the formulation contains 0.01-1.5 wt.% asiaticoside, even more preferably 0.02-0.6 wt.% asiaticoside and most preferably 0.03-0.45 wt.% asiaticoside.

The triterpenic genins selected from asiatic acid, madecassic acid and combinations thereof are preferably present in the formulation in a concentration of 0.01-2 wt.%, more preferably in a concentration of 0.03-1.5 wt.% and most preferably in a concentration of 0.05-1 wt.%.

In a preferred embodiment, the formulation of the present invention contains asiaticoside and triterpenic genins selected from asiatic acid, madecassic acid and combinations thereof in a weight ratio asiaticoside : triterpenic genins of 1:4 to 4:1, more preferably in a weight ratio asiaticoside : triterpenic genins of 1:1 to 1:2

Cannabidiol and the triterpenes are typically present in the formulation in a weight ratio of 10:1 to 1:10, more preferably in a weight ratio of 6:1. to 1:4, most preferably in a weight ratio of 4:1 to 1:1.

In accordance with another preferred embodiment, the formulation additionally contains hemp seed oil. More preferably, the formulation contains 0.01-5 wt.%, more preferably 0.03-3 wt.%, most preferably 0.1- 1 wt.% of hemp seed oil.

Hemp seed oil is obtained by pressing hemp seeds. Hemp seed oil is manufactured from varieties of *Cannabis sativa* that do not contain significant amounts of tetrahydrocannabinol. Typically around 50% of the weight of hempseed is an edible oil that contains omega-6 fatty acids including linoleic acid (appr. 54%) and gamma-linolenic acid (appr. 3%), as well as the omega-3 fatty acid alpha-linolenic acid (appr. 17%) in addition to monounsaturated fatty acids and stearidonic acid (appr. 2%). Hemp seed oil typically contains 5% to 7% saturated fatty acids.

The formulation of the present invention typically comprises 5-90 wt.% water. More preferably, the water content of the formulation is in the range of 10 to 85 wt.%, most preferably in the range of 20 to 80 wt.%.

The formulation of the present invention can be provided in different forms. Preferably, the formulation is a gel, a cream, a lotion, a soap or a spot treatment product.

Another aspect of the invention relates to a topical formulation comprising:
- cannabidiol;
- flavonolignans selected silibinin, isosilibinin, silicristin, silidianin and combinations thereof, the concentrations of these flavonolignans being calculated as silibinin;
- triterpenes selected from asiaticoside, madecassoside, asiatic acid, madecassic acid and combinations thereof.

Advantageous embodiments of this formulation have already been described above.

Yet another aspect of the invention relates to a method of preparing topical formulation as described herein before, said method comprising combining an extract of *Cannabis,* an extract of *S*. *marianum* and an extract of *C*. *asiatica.*

The extract of *Cannabis* that is employed in this method preferably contains at least 20%, more preferably at least 30% and most preferably at least 40% cannabidiol by weight of dry matter.

The extract of *S*. *marianum* that is used in the method preferably contains at least 20%, more preferably at least 30% and most preferably at least 40% of the flavonolignans (silibinin, isosilibinin, silicristin, silidianin and combinations thereof) by weight of dry matter.

The extract of *C*. *asiatica* used in the method preferably contains at least 20%, more preferably at least 30% and most preferably at least 40% of the triterpenes (asiaticoside, madecassoside, asiatic acid, madecassic acid and combinations thereof) by weight of dry matter.

The invention is further illustrated by the following non-limiting examples.

### EXAMPLES

### Example 1

The impact of cannabidiol, extract of *Silybum marianum* (fruit without pappus) and combinations of these components on the production of cytokines IL-1β and TNFα by a U937 cell line were investigated.

The specifications of the aforementioned components are provided in Table 1.

**Table 1**

| | **Supplier** | **Characteristics** |
|---|---|---|
| **Cannabidiol** | **Echo Pharmaceuticals BV, the Netherlands** | **Extracted Cannabidiol (98%)** |
| ***S*. *marianum* extract** | Indena S.p.a., Italy | Powder (bulk density 0.44 g/ml) 91 wt.% ≤ 50 µm |
| | Product code 9065110 | Extraction solvent: ethyl acetate |
| | | Flavonoids (as monohydrate Silibinin) ≥ 80.0% (UV assay) |
| | | Silibinin and Isosilibinin ≥ 30.0% (HPLC assay) |
| | | Silymarin (as Silibinin): 50.0 - 60.0% (HPLC assay) |
| | | Silicristin and Silidianin (as Silibinin), on the total content of Silymarin: 20.0 - 45.0% (HPLC assay) |
| | | Silibinin A and Silibinin B (as Silibinin), on the total content of Silymarin: 40.0 - 65.0% (HPLC assay) |
| | | Isosilibinin A and Isosilibinin B (as Silibinin), on the total content of Silymarin: 10.0 - 20.0% (HPLC assay) |
| | | Proteins: 1.7 wt.% |
| | | Fat: 2.3 wt. % |
| | | Dietary fibre: 3.5 wt.% |
| | | Moisture 1.4 wt.% |

### Inhibition of secretion of IL-1β and TNFα

U937 cells, were thawed and treated according to the manufacturer's instructions. The cultures were incubated for recovery at 37°C with 5% CO₂ until 70-80% confluency was reached (visual estimation). Then, approx. 0.3x10⁶¢/mL (determined by counting) were seeded in 96 well plates containing 200 µL/well of complete growth medium. The cells were incubated at 37°C with 5% CO₂ until 60% confluency was reached (visual estimation, typically - after 48hr).

After the cells had reached the required confluency, the medium was replaced with preprepared growth medium supplemented with 1 µg/mL of lipopolysaccharides, and the test components were added, at a final volume of 200 µL/well. The following control groups were also added: Naive cells, vehicle control, stimulation control, stimulation vehicle control. A proper blank control was subtracted from the measurements.

The cells were incubated for 24 hr. at 37°C with 5% CO₂. After the incubation, the conditioned medium of the different treatment groups was collected under standardized conditions and centrifuged at 250 x g for 5 min to remove particulates. Clear supernatants were frozen at -70°C until cytokines analyses. The secretion levels of IL-1β and TNFα (a major inflammatory marker) were quantified by commercial ELISA.

The results show (see Table 2) that:
- cannabidiol (CBD) attenuates secretion of IL-1β and TNFα
- extract of *S*. *Marianum* attenuates secretion of IL-1β and *TNFα*
- the combination of CBD and extract of *S*. *Marianum* has a synergistic inhibitory effect on the secretion of IL-1β and *TNFα*

### Results

The results of the aforementioned investigations are summarised in Table 2.

**Table 2**

| | **µg/ml** | | **Inhibition (in %)** | |
|---|---|---|---|---|
| **Sample** | **Cannabidiol** | ***S*. *Marianum*** | **IL-1β** | **TNFα** |
| 1 | 10 | 0 | 28.4 | 48.0 |
| 2 | 0 | 10 | 0 | 0 |
| 3 | 10 | 10 | 40.9 | 60 |

### Example 2

The impact of cannabidiol, extract of *Silybum marianum* (fruit without pappus) and extract of *Centella asiatica* (leaf) on the secretion of prostaglandin E2 (PGE₂) was investigated.

The cannabidiol and *S*. *marianum* extract used were the same as in Example 1. The specification of the *C*. *asiatica* extract is shown in Table 3.

**Table 3**

| | **Supplier** | **Characteristics** |
|---|---|---|
| ***C*. *asiatica* extract** | Indena S.p.a., Italy | Powder (bulk density 0.40 g/ml) |
| | | 80 wt.% ≤ 50 µm |
| | Product code 3022060 | Extraction solvent: ethanol |
| | | Asiaticoside: 36.0-44.0% (HPLC assay) |
| | | Sum of Asiatic acid and Madecassic aid: |
| | | 56.0-64.0% (HPLC assay) |
| | | Proteins: 1.4 wt.% |
| | | Fat: 0.4 wt. % |
| | | Moisture 1.0 wt.% |

### Secretion of PGE₂

RAW 264.7 cells (approx. 2.5x10⁵¢/ml, by counting) were seeded in 96 well plates containing 170 µl/well of complete growth medium. The cells were incubated at 37°C with 5% CO₂ for 24 hr. Then, the medium was aspirated and replaced by LPS-containing medium (12.5 ng/ml) without or with the test components. In addition, naive cells, vehicle-treated cells, Stimulated Control, and Stimulated Vehicle Control served as negative controls. Dexamethasone served as a positive control for anti-inflammatory assay. A Blank control group was included in the assay.

The spent media from all test groups were collected under standardized conditions and centrifuged at 250g for 5 min to remove particulates. Clear supernatants were frozen at - 70°C until analysis. The secretion levels of PGE₂ (an important inflammatory marker) were determined.

### Results

The results of the aforementioned investigation are summarised in Table 4.

**Table 4**

| | **µg/ml** | | | **Inhibition (in %)** |
|---|---|---|---|---|
| **Sample** | **Cannabidiol** | ***S*. *marianum*** | ***C. asiatica*** | ***PGE₂*** |
| 1 | 2.5 | 0 | 0 | 24 |
| 2 | 0 | 2.5 | 0 | 5.0 |
| 3 | 0 | 0 | 2.5 | 2.4 |
| 4 | 2.5 | 2.5 | 2.5 | 46 |

## Claims

1. A topical formulation for use in a method of treating or preventing an inflammatory skin condition, said method comprising topically administrating a formulation comprising (i) 0.1-3 wt.% cannabidiol and (ii) 0.05-2 wt.% of flavonolignans selected from silibinin, isosilibinin, silicristin, silidianin and combinations thereof, the concentrations of these flavonolignans being calculated as silibinin.

2. Topical formulation for use in a method of treating or preventing an inflammatory skin condition according to claim 1, wherein the formulation contains 0.3-2 wt.% cannabidiol.

3. Topical formulation for use in a method of treating or preventing an inflammatory skin condition according to claim 1 or 2, wherein the formulation contains 0.25-1 wt.% of the flavonolignans, the concentrations of these flavonolignans being calculated as silibinin.

4. Topical formulation for use in a method of treating or preventing an inflammatory skin condition according to any one of the preceding claims, wherein the formulation contains cannabidiol and the flavonolignans in a weight ratio of 1:5 to 5:1.

5. Topical formulation for use in a method of treating or preventing an inflammatory skin condition according to any one of the preceding claims, wherein the formulation contains an extract of *Silybum marianum.*

6. Topical formulation for use in a method of treating or preventing an inflammatory skin condition according to any one of the preceding claims, wherein the inflammatory skin condition is a sebaceous gland disorder selected from acne, seborrhea, rosacea, perioral dermatitis, corticosteroid-induced acneiform lesions and oily skin, preferably the inflammatory skin condition is acne.

7. A topical formulation comprising:
• cannabidiol;
• flavonolignans selected from silibinin, isosilibinin, silicristin, silidianin and combinations thereof, the concentrations of these flavonolignans being calculated as silibinin;
• triterpenes selected from asiaticoside, madecassoside, asiatic acid, madecassic acid and combinations thereof.

8. Topical formulation according to claim 7, wherein the formulation contains 0.1-3 wt.% cannabidiol.

9. Topical formulation according to claim 7 or 8 wherein the formulation contains 0.05-2 wt.% of the flavonolignans.

10. Topical formulation according to any one of claims 7-9, the formulation contains 0.025-3 wt.% of the triterpenes.

11. Topical formulation according to any one of claims 7-10, wherein the formulation contains cannabidiol and the flavonolignans in a weight ratio of 1:5 to 5:1.

12. Topical formulation according to any one of claims 7-11, wherein the formulation contains cannabidiol and the triterpenes in a weight ratio of 10:1 to 1:2.

13. Topical formulation according to any one of claims 7-12, wherein the formulation contains an extract of *Silybum marianum.*

14. Topical formulation according to any one of claims 7-13, wherein the formulation contains an extract of *Centella* asiatica.

15. A method of preparing topical formulation according to any one claims 7-14, said method comprising combining an extract of *Cannabis,* an extract of *Silybum marianum* and an extract of *Centella asiatica.*

## Patentansprüche

1. Topische Formulierung zur Verwendung in einem Verfahren zum Behandeln oder Vorbeugen einer entzündlichen Hauterkrankung, wobei das besagte Verfahren das topische Verabreichen einer Formulierung umfasst, die (i) 0,1-3 Gew.-% Cannabidiol und (ii) 0,05-2 Gew.-% Flavonolignane enthält, die aus Silibinin, Isosilibinin, Silicristin, Silidianin und Kombinationen davon ausgewählt sind, wobei die Konzentrationen dieser Flavonolignane als Silibinin berechnet werden.

2. Topische Formulierung zur Verwendung in einem Verfahren zum Behandeln oder Vorbeugen einer entzündlichen Hauterkrankung nach Anspruch 1, wobei die Formulierung 0,3-2 Gew.-% Cannabidiol enthält.

3. Topische Formulierung zur Verwendung in einem Verfahren zum Behandeln oder Vorbeugen einer entzündlichen Hauterkrankung nach Anspruch 1 oder 2, wobei die Formulierung 0,25-1 Gew.-% der Flavonolignane enthält, wobei die Konzentrationen dieser Flavonolignane als Silibinin berechnet werden.

4. Topische Formulierung zur Verwendung in einem Verfahren zum Behandeln oder Vorbeugen einer entzündlichen Hauterkrankung nach einem der vorhergehenden Ansprüche, wobei die Formulierung Cannabidiol und die Flavonolignane in einem Gewichtsverhältnis von 1:5 bis 5:1 enthält.

5. Topische Formulierung zur Verwendung in einem Verfahren zum Behandeln oder Vorbeugen einer entzündlichen Hauterkrankung nach einem der vorhergehenden Ansprüche, wobei die Formulierung einen Extrakt aus *Silybum marianum* enthält.

6. Topische Formulierung zur Verwendung in einem Verfahren zum Behandeln oder Vorbeugen einer entzündlichen Hauterkrankung nach einem der vorhergehenden Ansprüche, wobei die entzündliche Hauterkrankung eine Talgdrüsenstörung ist, die aus Akne, Seborrhoe, Rosazea, perioraler Dermatitis, Kortikosteroid-induzierten akneiformen Läsionen und fettiger Haut ausgewählt ist, wobei die entzündliche Hauterkrankung vorzugsweise Akne ist.

7. Topische Formulierung, die Folgendes enthält:
• Cannabidiol;
• Flavonolignane, die aus Silibinin, Isosilibinin, Silicristin, Silidianin und Kombinationen davon ausgewählt sind, wobei die Konzentrationen dieser Flavonolignane als Silibinin berechnet werden;
• Triterpene, die aus Asiaticosid, Madecassosid, Asiatinsäure, Madecassinsäure und Kombinationen davon ausgewählt sind.

8. Topische Formulierung nach Anspruch 7, wobei die Formulierung 0,1-3 Gew.-% Cannabidiol enthält.

9. Topische Formulierung nach Anspruch 7 oder 8, wobei die Formulierung 0,05-2 Gew.-% der Flavonolignane enthält.

10. Topische Formulierung nach einem der Ansprüche 7-9, wobei die Formulierung 0,025-3 Gew.-% der Triterpene enthält.

11. Topische Formulierung nach einem der Ansprüche 7-10, wobei die Formulierung Cannabidiol und die Flavonolignane in einem Gewichtsverhältnis von 1:5 bis 5:1 enthält.

12. Topische Formulierung nach einem der Ansprüche 7-11, wobei die Formulierung Cannabidiol und die Triterpene in einem Gewichtsverhältnis von 10:1 bis 1:2 enthält.

13. Topische Formulierung nach einem der Ansprüche 7-12, wobei die Formulierung einen Extrakt aus *Silybum marianum* enthält.

14. Topische Formulierung nach einem der Ansprüche 7-13, wobei die Formulierung einen Extrakt aus *Centella asiatica* enthält.

15. Verfahren zum Herstellen einer topischen Formulierung nach einem der Ansprüche 7-14, wobei das besagte Verfahren das Kombinieren eines Extrakts aus Cannabis, eines Extrakts aus *Silybum marianum* und eines Extrakts aus *Centella asiatica* umfasst.

## Revendications

1. Formulation topique destinée à être utilisée dans un procédé de traitement ou de prévention d'une affection cutanée inflammatoire, ledit procédé comprenant l'administration topique d'une formulation comprenant (i) 0,1 à 3 % en poids de cannabidiol et (ii) 0,05 à 2 % en poids de flavonolignans sélectionnés parmi la silibinine, l'isosilibinine, la silicristine, la silidianine et leurs combinaisons, les concentrations de ces flavonolignanes étant calculées comme silibinine.

2. Formulation topique destinée à être utilisée dans un procédé de traitement ou de prévention d'une affection cutanée inflammatoire selon la revendication 1, où la formulation contient 0,3 à 2 % en poids de cannabidiol.

3. Formulation topique destinée à être utilisée dans un procédé de traitement ou de prévention d'une affection cutanée inflammatoire selon la revendication 1 ou 2, où la formulation contient 0,25 à 1 % en poids de flavonolignanes, les concentrations de ces flavonolignanes étant calculées comme silibinine.

4. Formulation topique destinée à être utilisée dans un procédé de traitement ou de prévention d'une affection cutanée inflammatoire selon l'une quelconque des revendications précédentes, où la formulation contient du cannabidiol et les flavonolignanes dans un rapport pondéral de 1:5 à 5:1.

5. Formulation topique destinée à être utilisée dans un procédé de traitement ou de prévention d'une affection cutanée inflammatoire selon l'une quelconque des revendications précédentes, où la formulation contient un extrait de *Silybum marianum.*

6. Formulation topique destinée à être utilisée dans un procédé de traitement ou de prévention d'une affection cutanée inflammatoire selon l'une quelconque des revendications précédentes, où l'affection cutanée inflammatoire est un trouble des glandes sébacées choisi parmi l'acné, la séborrhée, la rosacée, la dermatite périorale, les lésions acnéiformes induites par les corticostéroïdes et la peau grasse, de préférence l'affection cutanée inflammatoire est l'acné.

7. Formulation topique comprenant :
• du cannabidiol ;
• des flavonolignanes choisis parmi la silibinine, l'isosilibinine, la silicristine, la silidianine et leurs combinaisons, les concentrations de ces flavonolignanes étant calculées comme silibinine ;
• des triterpènes choisis parmi l'asiaticoside, le madécassoside, l'acide asiatique, l'acide madécassique et leurs combinaisons.

8. Formulation topique selon la revendication 7, où la formulation contient 0,1 à 3 % en poids de cannabidiol.

9. Formulation topique selon la revendication 7 ou 8, où la formulation contient 0,05 à 2 % en poids des flavonolignanes.

10. Formulation topique selon l'une quelconque des revendications 7 à 9, où la formulation contient 0,025 à 3 % en poids des triterpènes.

11. Formulation topique selon l'une quelconque des revendications 7 à 10, où la formulation contient du cannabidiol et les flavonolignanes dans un rapport pondéral de 1:5 à 5:1.

12. Formulation topique selon l'une quelconque des revendications 7 à 11, où la formulation contient du cannabidiol et les triterpènes dans un rapport pondéral de 10:1 à 1:2.

13. Formulation topique selon l'une quelconque des revendications 7 à 12, où la formulation contient un extrait de *Silybum marianum.*

14. Formulation topique selon l'une quelconque des revendications 7 à 13, où la formulation contient un extrait de *Centella asiatica.*

15. Procédé de préparation d'une formulation topique selon l'une quelconque des revendications 7 à 14, ledit procédé comprenant la combinaison d'un extrait de *Cannabis,* d'un extrait de *Silybum marianum* et d'un extrait de *Centella asiatica.*
